# EUROPEAN PATENT SPECIFICATION

(11) **EP 1 608 311 B1**
(45) Date of publication and mention of the grant of the patent: **28.07.2010**
(21) Application number: 04722129.6
(22) Date of filing: 19.03.2004
(51) Int. Cl.: A61K 31/397, A61P 3/06, A61K 45/06

(54) **USE OF LOW MOLECULAR WEIGHT THROMBIN INHIBITORS IN CHOLESTEROL-LOWERING THERAPY**
VERWENDUNG VON NIEDERMOLEKULAREN THROMBIN-HEMMERN IN DER CHOLESTERINSENKENDEN THERAPIE
UTILISATION D'INHIBITEURS DE THROMBINE DE FAIBLE POIDS MOLECULAIRE DANS UN TRAITEMENT HYPOCHOLESTEROLEMIANT

(30) Priority: 22.03.2003 GB 0306615
(43) Date of publication of application: 28.12.2005
(73) Proprietor: AstraZeneca AB, 151 85 Södertälje (SE)
(72) Inventor: GRIND, Margaretha, AstraZeneca R & D Charnwood, Loughborough, Leicestershire LE11 5RH (GB)
(86) International application number: PCT/SE2004/000417
(87) International publication number: WO 2004/082702

(56) References cited:
- EP-A2- 0 432 537
- WO-A-01/95931
- WO-A-02/36157
- WO-A-02/44145
- WO-A-97/40024
- WO-A1-98/11896
- MARTINDALE: "The complete Drug Reference, 34th Edition" PHARMACEUTICAL PRESS * page 811 * * page 823 - page 825 *

## Description

### Field of the Invention

This invention relates to a new use of low molecular weight thrombin inhibitors.

### Background and Prior Art

It is well known that high levels of cholesterol are associated with heart disease. More than half of all US citizens are understood to have cholesterol levels that exceed those recommended, and one in five has cholesterol levels that are considered high.

Cholesterol is involved in the production and maintenance of cell membranes, as well as the production of sex hormones (including progesterone, testosterone, estradiol and cortisol), bile salts and Vitamin D. It is formed primarily in the liver, but also in other parts of the body, such as the small intestine.

In healthy individuals, all of the cholesterol that is needed to perform the above-mentioned functions is produced naturally. However, in a typical blood test, of the amount of cholesterol circulating in blood, about 85% is endogenous, the other 15% arising from external sources. Dietary cholesterol usually originates from meat, poultry, fish, seafood and dairy products. In this respect, high consumption levels of these foodstuffs may give rise to increased cholesterol levels in the bloodstream.

Increased cholesterol levels in serum have been associated with atherosclerosis, which is known to increase significantly the risk of blood vessel blockage (stenosis), and thus the likelihood of angina pectoris, myocardial infarction and other cardiovascular complications, such as stroke.

Cholesterol is insoluble in aqueous environments and thus needs to be transported within the bloodstream by apolipoproteins (Apos). When apolipoproteins are associated with cholesterol, complexes known as lipoproteins are formed. The density of these lipoproteins is determined by the amount of protein in the molecule and, in this respect, low-density lipoproteins (LDLs), which are the major cholesterol carrier in the blood, are known to have more of the negative effects mentioned herein than protective high-density lipoproteins (HDLs). High levels of LDLs are thus associated with atherosclerosis, whereas greater levels of HDLs are understood to provide some protection against stenosis, and hence coronary risk, by way of removal of excess cholesterol (transporting it to the liver for disposal).

A third group of carrier molecules, very low-density lipoproteins (VLDLs) are converted to LDLs following the delivery of triglycerides to the muscles and adipose tissue. Triglycerides are a mixture of fatty acids and glycerol and are the major components of lipids circulating in blood. Like cholesterol, triglycerides are substances that are found endogenously in the bloodstream, and may be deposited in adipose tissue. Triglycerides contain high-energy fatty acids which provide much of the fuel needed for normal cellular function. However, an excessive amount of triglycerides, or VLDLs, in the bloodstream can result in similar problems to those associated with high cholesterol and LDL levels, as well as obesity and diabetes.

Thus, levels of HDLs, LDLs, total cholesterol and triglycerides are all key indicators in determining the risk of atherosclerosis and associated cardiovascular disorders, such as coronary artery diseases (e.g. angina pectoris, myocardial infarction, etc.), stroke (including cerebro-vascular accident and transient ischaemic attack), peripheral arterial occlusive disease, obesity and diabetes. Patients with high overall cholesterol and/or triglycerides levels are at a significant risk, irrespective of whether or not they also have a favourable HDL level. Patients with normal cholesterol levels but low HDL levels are also at increased risk. Recently, it has also been noted that the level of risk of cardiovascular disease associated with high levels of apolipoprotein B (ApoB; which carries lipids in VLDLs and LDLs), and/or low levels of apolipoprotein A-I (ApoA-1; which carries lipids in HDLs), is extremely high.

There are numerous factors that influence cholesterol and triglyceride levels, including diet, age, weight, gender, genetics, diseases (such as diabetes) and lifestyle.

Positive changes in relation to diet, lifestyle and exercise are often insufficient to decrease the risk of cardiovascular problems. In such instances, cholesterol- and/or triglyceride-lowering medication may be prescribed.

Drugs that reduce LDL levels in serum can prevent or reduce the build-up of artery blocking plaques, and can reduce the risk of plaque rupture and associated thrombo-embolic complications. There are several types of drugs that can help reduce blood cholesterol levels. The most commonly prescribed are the statins, HMG-CoA reductase inhibitors, such as lovastatin, pravastatin, fluvastatin, simvastatin, atorvastatin, pitavastatin and rosuvastatin (e.g. rosuvastatin-calcium). These drugs prevent directly the formation of cholesterol in the liver and thus reduce the risk of cardiovascular disease. Other prescribed drug categories include resins (such as cholestyramine and colestipol), which act by binding bile acids, so causing the liver to produce more of the latter, and using up cholesterol in the process. Further, the B vitamin niacin has been reported at high doses to lower triglycerides and LDL levels in addition to increasing HDL levels. Fibrates (such as gemfibrozil and fenofibrate) are known to lower triglycerides and can increase HDL levels.

However, some of these drugs are known to have side effects, including liver damage. Hence, there is a need for alternative and/or more effective drugs for use in cholesterol-lowering therapy.

The early development of low molecular weight inhibitors of thrombin has been described by Claesson in Blood Coagul. Fibrinol. (1994) 5, 411. Low molecular weight thrombin inhibitors (and prodrugs thereof) have been described more recently in US Patent N° 4,346,078; International Patent Applications WO 93/11152, WO 93/18060, WO 93/05069, WO 94/20467, WO 94/29336, WO 95/35309, WO 95/23609, WO 96/03374, WO 96/06832, WO 96/06849, WO 96/17860, WO 96/24609, WO 96/25426, WO 96/32110, WO 97/01338, WO 97/02284, WO 97/15190, WO 97/23499, WO 97/30708, WO 97/40024, WO 97/46577, WO 98/06740, WO 97/49404, WO 97/11693, WO 97/24135, WO 97/47299, WO 98/01422, WO 98/57932, WO 99/29664, WO 98/06741, WO 99/37668, WO 99/37611, WO 98/37075, WO 99/00371, WO 99/28297, WO 99/29670, WO 99/40072, WO 99/54313, WO 96/31504, WO 00/01704, WO 00/08014, WO 00/35859, WO 00/35869, WO 00/42059, WO 00/61577, WO 00/61608, WO 00/61609, WO 01/87879, WO 02/14270, WO 02/44145, WO 03/000653, WO 04/000818 and WO 04/014894; and European Patent Applications 648 780, 468 231, 559 046, 641 779, 185 390, 526 877, 542 525, 195 212, 362 002, 364 344, 530 167, 293 881, 686 642, 669 317, 601 459 and 623 596.

In particular, international patent application WO 94/29336 discloses a group of compounds, including HOOC-CH₂-(*R*)Cgl-(*S*)Aze-Pab-H (in which Cgl represents cyclohexylglycyl, Aze represents azetidine-2-carboxyl and Pab represents 4-amidinobenzylamino), which is also known as melagatran (see Example 1 of WO 94/29336). International Patent Application WO 97/23499 discloses prodrugs of *inter alia* melagatran.

More recently, international patent application WO 02/44145 discloses α-hydroxy acid-based low molecular weight thrombin inhibitors and prodrugs thereof.

To the applicant's knowledge, none of the above-mentioned documents disclose or suggest the direct use a low molecular weight thrombin inhibitor or a prodrug thereof in cholesterol-lowering therapy and/or modifications of lipid (including triglyceride), lipoprotein, or apolipoprotein, profiles.

### Disclosure of the Invention

We have found, surprisingly, that administration of a low molecular weight thrombin inhibitor may give rise to reduced levels of lipids, such as total cholesterol, LDLs (i.e. LDL-cholesterol) and triglycerides in the bloodstream, in addition to increasing HDL (i.e. HDL-cholesterol) levels.

According to a first aspect of the invention there is provided the use of a low molecular weight thrombin inhibitor as defined hereinafter, or a pharmaceutically acceptable derivative thereof as defined hereinafter, for the manufacture of a medicament for use in cholesterol-lowering therapy.

When employed in the context of the present invention and disclosure, the term "cholesterol-lowering therapy" includes any therapy that results in beneficial modifications of serum profiles of total cholesterol, lipids (including triglycerides), lipoproteins or apolipoproteins, and will thus be understood to encompass the terms "lipid-modifying therapy" and "lipid-(and triglyceride-) lowering therapy", as well as the treatment of hyperlipidaemias (i.e. the elevation of lipids in the bloodstream), including hypercholesterolaemia (high cholesterol levels in the blood; including primary and secondary (combined) hypercholesterolaemia), hyperlipoproteinemia (elevate plasma lipoproteins levels) and/or hypertriglyceridemia (high triglycerides levels in the blood). The term will thus be understood to include types I, II (IIa and IIb), III, IV and/or V hyperlipoproteinaemia, as well as secondary hypertriglyceridaemia and/or familial lecithin cholesterol acyltransferase deficiency, but in principle includes any treatment of a patient which results in a decrease in serum levels of cholesterol, LDLs, VLDLs, triglycerides and/or ApoB, and/or an increase in serum levels of DLs and/or ApoA-I.

For the avoidance of doubt, in the context of this disclosure, the terms "treatment", and "therapy" include the therapeutic and/or prophylactic treatment of patients in need of modifications of cholesterol, lipid (including triglyceride), lipoprotein and/or apolipoprotein profiles.

"Pharmaceutically acceptable derivatives" of claimed the thrombin inhibitors refers to salts (e.g. pharmaceutically-acceptable non-toxic organic or inorganic acid addition salts) and solvates.

Low molecular weight peptide-based thrombin inhibitors that may be used in accordance with the invention include HOOC-CH₂-*(R)*Cgl-*(S)*Aze-Pab-H (known as melagatran; see above and International Patent Application WO 94129336), and compounds of the formula I, 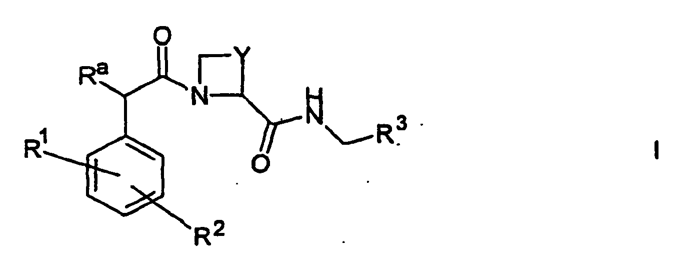 wherein
R^{a} represents -OH or -CH₂OH;
R¹ represents at least one optional halo substituent;
R² represents one or two C₁₋₃ alkoxy substituents, the alkyl parts of which substituents are themselves substituted with one or more fluoro substituents (i.e. R² represents one or two fluoroalkoxy(C₁₋₃) groups);
Y represents -CH₂- or -(CH₂)₂-; and
R³ represents a structural fragment of formula I(i) or I(ii): 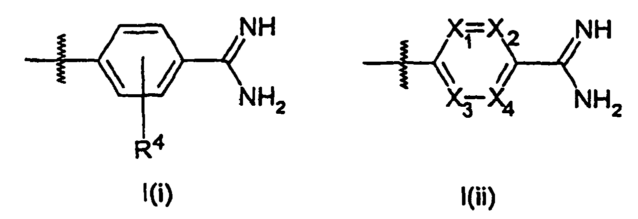 wherein
R⁴ represents H or one or more fluoro substituents; and one or two of X₁, X₂, X₃ and X₄ represent -N- and the others represent -CH-.

Preferred compounds of formula I include:
(a) Ph(3-Cl)(5-OCHF₂)-(*R*)CH(OH)C(O)-(*S*)Aze-Pab-H: 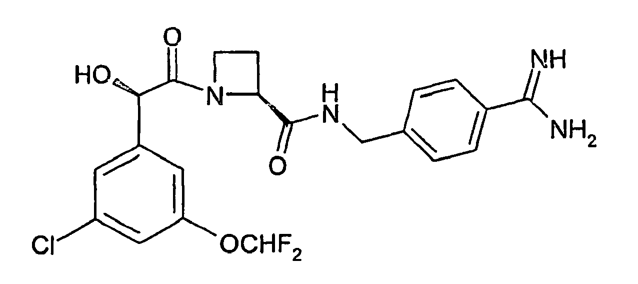
(b) Ph(3-Cl)(5-OCHF2)-(*R*)CH(OH)C(O)-(*S*)Aze-Pab(2,6-diF): 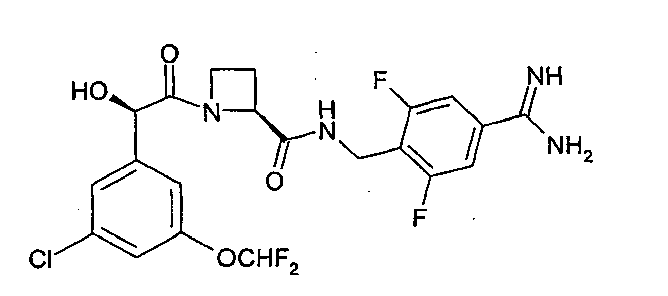
(c) Ph(3-Cl)(5-OCH₂CH₂F)-(*R*)CH(OH)C(O)-(*S*)Aze-Pab-H:

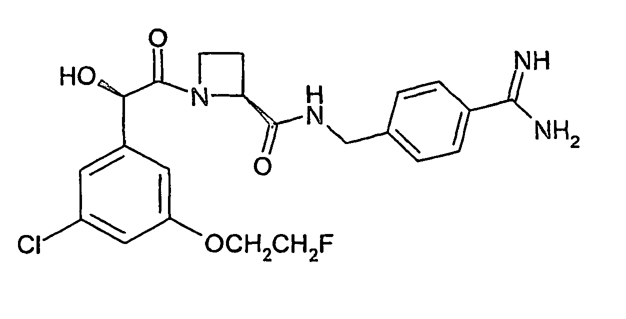

Also included are compounds that are known to act as prodrugs of the thrombin inhibitor melagatran of the formula R¹O₂C-CH₂-(*R*)Cgl-*(S)*Aze-Pab-OH (see the list of abbreviations above or in WO 97/23499), wherein R¹ represents C₁₋₁₀ alkyl or benzyl, such as linear or branched C₁₋₆ alkyl (e.g. C₁₋₄ alkyl, especially methyl, propyl and, particularly, ethyl) and the OH group replaces one of the amidino hydrogens in Pab.

Also included are compounds that act as prodrugs of compounds of formula I which are compounds of formula Ia, 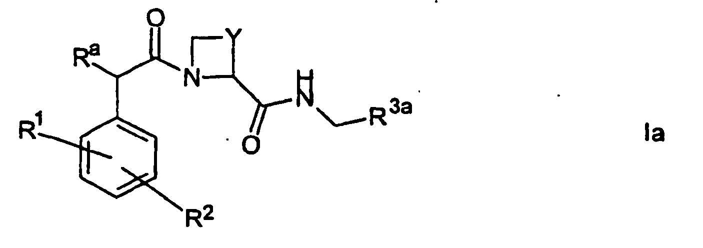 wherein R^{3a} represents a structural fragment of formula I(iii) or I(iv): 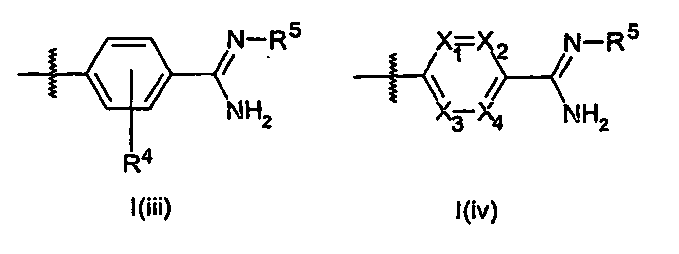
wherein R⁵ represents OR⁶ or C(O)OR⁷;
R⁶ represents H, C₁₋₁₀ alkyl, C₁₋₃ alkylaryl or C₁₋₃ alkyloxyaryl, the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents;
R⁷ represents C₁₋₁₀ alkyl which latter group is optionally interrupted by one or more oxygen atoms, or C₁₋₃ alkylaryl or C₁₋₃ alkyloxyaryl, the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents), and
R^{a}, R¹, R², Y, R⁴, X₁, X₂, X₃ and X₄ are as hereinbefore defined.

Hence preferred compounds of formula Ia include:
(i) Ph(3-Cl)(5-OCHF₂-*(R)*CH(OH)C(O)-(*S*)Aze-Pab(OMe): 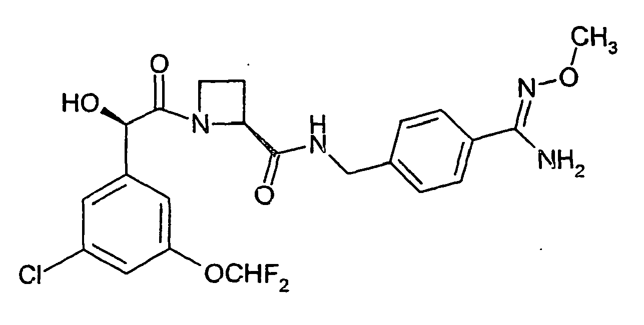
(ii) Ph(3-Cl)(5-OCHF₂)-(*R*)CH(OH)C(O)-(*S*)Aze-Pab(2,6-diF)(OMe): 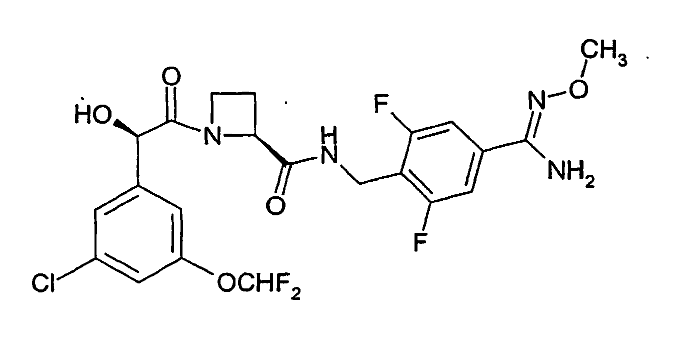
(iii) Ph(3-Cl)(5-OCH₂CH₂F)-(*R*)CH(OH)C(O)-(*S*)Aze-Pab(OMe):

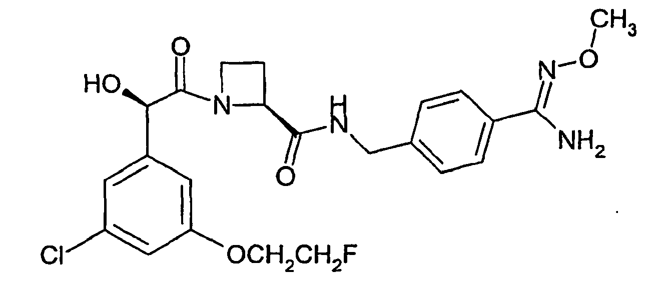

Compounds of formulae I and Ia may be made in accordance with techniques described in international patent application WO 02/44145.

In accordance with the invention, thrombin inhibitors and derivatives thereof of the present invention may be administered orally, intravenously, subcutaneously, buccally, rectally, dermally, nasally, tracheally, bronchially, topically, by any other parenteral route, or *via* inhalation, in the form of a pharmaceutical preparation comprising the thrombin inhibitor or prodrug as defined above in a pharmaceutically acceptable dosage form. Depending on the disorder, and the patient, to be treated, as well as the route of administration, the compositions may be administered at varying doses.

Preferred modes of delivery are systemic. For melagatran and derivatives thereof of the present invention, preferred modes of administration are parenteral, more preferably intravenous, and especially subcutaneous. For prodrugs of melagatran as defined above and compounds of formula Ia, preferred modes of administration are oral.

In the therapeutic treatment of mammals, and especially humans, the presently claimed thrombin inhibitors, prodrugs of thrombin inhibitors, and derivatives of either will generally be administered as a pharmaceutical formulation in admixture with a pharmaceutically acceptable adjuvant, diluent or carrier, which may be selected with due regard to the intended route of administration and standard pharmaceutical practice.

Suitable formulations for use in administering thrombin inhibitors are known in the art, and include those known from US Patent N° 4,346,078; International Patent Applications WO 93/11152, WO 93/18060, WO 93/05069, WO 94/20467, WO 94/29336, WO 95/35309, WO 95/23609, WO 96/03374, WO 96/06832, WO 96/06849, WO 96/17860, WO 96/24609, WO 96/25426, WO 96/32110, WO 97/01338, WO 97/02284, WO 97/15190, WO 97/30708, WO 97/40024, WO 97/46577, WO 98/06740, WO 97/49404, WO 97/11693, WO 97/24135, WO 97/47299, WO 98/01422, WO 98/57932, WO 99/29664, WO 98/06741, WO 99/37668, WO 99/37611, WO 98/37075, WO 99/00371, WO 99/28297, WO 99/29670, WO 99/40072, WO 99154313, WO 96/31504, WO 00/01704, WO 00/08014, WO 00/35859, WO 00/35869, WO 00/42059, WO 00/61577, WO 00/61E08, WO 00/61609, WO 01/37879, WO 02/14270, WO 02/44145, WO 03/000653 and WO 04/000818; and European Patent Applications 648 780, 468 231, 559 046, 641 779, 185 390, 526 877, 542 525, 195 212, 362 002, 364 344, 530 167, 293 881, 686 642, 669 317, 601 459 and 623 596.

Suitable formulations for use with melagatran, derivatives and prodrugs thereof as defined above are described in the literature, for example as described in *inter alia* international patent applications WO 94/29336, WO 96/14084, WO 96/16671, WO 97/23499, WO 97/39770, WO 97145138, WO 98/16252, WO 99/27912, WO 99/27913, WO 00/12043 and WO 00/13671. Otherwise, the preparation of suitable formulations may be achieved non-inventively by the skilled person using routine techniques.

The amounts of thrombin inhibitor or derivative in the formulation will depend on the severity of the condition, and on the patient, to be treated, as well as the compound(s) which is/are employed, but may be determined non-inventively by the skilled person.

Suitable doses of the presently claimed thrombin inhibitors and derivatives thereof in the therapeutic and/or prophylactic treatment of mammalian, especially human, patients may be determined routinely by the medical practitioner or other skilled person, and include the respective doses discussed in the relevant prior art documents that are mentioned hereinbefore.

In the case of melagatran, suitable doses of active compound, prodrugs and derivatives thereof as presently claimed, in the therapeutic and/or prophylactic treatment of mammalian, especially human, patients include those which give a mean plasma concentration of up to 5 µmol/L, for example in the range 0.001 to 5 µmol/L over the course of treatment of the relevant condition. Suitable doses may thus be in the range 0.1 mg once daily to 25 mg three times daily, and/or up to 100 mg infuses parenterally over a 24 hour period, for melagatran, and in the range 0.1 mg once daily to 100 mg three times daily for prodrugs of melagatran as presently claimed see also the specific doses mentioned hereinafter for the prodrug of melagatran, ximelagatran).

In the case of compounds of formulae I and Ia, suitable daily doses of compounds in the therapeutic treatment of humans are 0.001-100 mg/kg body weight at peroral administration and 0.001-50 mg/kg body weight at parenteral administration.

In any event, the physician, or the skilled person, will be able to determine the actual dosage which will be most suitable for an individual patient, which is likely to vary with the severity of the condition that is to be treated, as well as the age, weight, sex and response of the particular patient to be treated. The above-mentioned dosages are exemplary of the average case; there can, of course, be individual instances where higher or lower dosage ranges are merited, and such are within the scope of this invention.

Low molecular weight thrombin inhibitors of the invention may be employed by way of co-administration along with other cholesterol-lowering, or lipid-lowering/modifying, drugs/therapies that are mentioned hereinbefore, such as the statins (HMG-CoA reductase inhibitors) and particularly any one of those statins specifically mentioned hereinbefore, in combination therapy.

According to a further aspect of the invention, there is provided a combination product comprising:
(A) a low molecular weight thrombin inhibitor as hereinbefore defined, or a pharmaceutically-acceptable derivative as hereinbefore defined; and
(B) another cholesterol-lowering, or lipid-lowering/modifying, therapeutic agent,
wherein each of components (A) and (B) is formulated in admixture with a pharmaceutically-acceptable adjuvant, diluent or carrier.

Such combination products provide for the administration of low molecular weight thrombin inhibitor/derivative in conjunction with the other therapeutic agent, and may thus be presented either as separate formulations, wherein at least one of those formulations comprises thrombin inhibitor/derivative, and at least one comprises the other therapeutic agent, or may be presented (i.e. formulated) at a combined preparation (i.e. presented as a single formulation including thrombin inhibitor/derivative and the other therapeutic agent).

Thus, there is further provided:
(1) a pharmaceutical formulation including a low molecular weight thrombin inhibitor as hereinbefore defined, or a pharmaceutically-acceptable derivative as hereinbefore defined another cholesterol-lowering, or lipid lowering/modifying, therapeutic agent; and a pharmaceutically-acceptable adjuvant, diluent or carrier; and
(2) a kit of parts comprising components:
   (a) a pharmaceutical formulation including, a low molecular weight thrombin inhibitor as hereinbefore defined, or a pharmaceutically-acceptable derivative as hereinbefore defined, in admixture with a pharmaceutically-acceptable adjuvant, diluent or carrier, and
   (b) a pharmaceutical formulation including another cholesterol-lowering, or lipid lowering/modifying, therapeutic agent in admixture with a pharmaceutically-acceptable adjuvant, diluent or carrier,
which components (a) and (b) are each provided in a form that is suitable for administration in conjunction with the other.

The composition described herein may have the advantage that, in cholesterol-lowering therapy, it may be more convenient for the physician and/or patient than, be more efficacious than, be less toxic than, have a broader range of activity than, be more potent than, produce fewer side effects than, or that it may have other useful pharmacological properties over, similar compositions known in the prior art for use in such therapy.

The invention is illustrates by the following examples, in which:
Figure 1 illustrates the difference in mean values (with 95% Confidence Intervals) of total cholesterol levels in serum as between patients receiving ximelagatran (36 mg bid) or warfarin (INR levels between 2 and 3) during the course of a clinical trial over a 21 month period.
Figure 2 illustrates the difference in mean values (with 95% Confidence Intervals) of levels of total triglycerides in serum as between patients receiving ximelagatran (36 mg bid) or warfarin (INR levels between 2 and 3) during the course of a clinical trial over a 21 month period.
Figure 3 illustrates the difference in mean values (with 95% Confidence Intervals) of LDL (i.e. LDL-cholesterol) levels in serum as between patients receiving ximelagatran (36 mg bid) or warfarin (INR levels between 2 and 3) during the course of a clinical trial over a 21 month period.
Figure 4 illustrates the difference in mean values (with 95% Confidence Intervals) of HDL (i.e. HDL-cholesterol) levels in serum as between patients receiving ximelagatran (36 mg bid) or warfarin (INR levels between 2 and 3) during the course of a clinical trial over a 21 month period.

### Example 1

### Lipid Measurements in Patients Undergoing Thrombin Inhibition Therapy in a Clinical Trial

A large-scale Phase III clinical trial was set up to establish the efficacy of the study compound EtO₂C-CH₂-(*R*)Cgl-(*S*)Aze-Pab-OH (ximelagatran; see Example 17 of international patent application WO 97/23499) in the prevention of stroke in patients with non-valvular atrial fibrillation, as compared to the current frontline treatment for this indication, warfarin.

Ximelagatran is a prodrug of the low molecular weight thrombin inhibitor, melagatran (see Example 1 of international patent application WO 94/29226).

The clinical trial protocol was similar to that described in international patent application WO 02/36157, with the following major differences:
(a) the study objective was to show that the efficacy of ximelagatran is non-inferior to that of dose-adjusted warfarin, aiming for an INR 2.0-3.0 (with INR measurements taken at least every 28 ± 3 days), in the prevention of all strokes (fatal and non-fatal) and systemic embolic events in patients with chronic non-valvular atrial fibrillation;
(b) the dosage of ximelagatran was fixed at 36 mg bid;
(c) in the exclusion criteria, subjects who had experienced stroke within the previous 30 days or transient ischaemic attack within the previous 3 days were excluded (as opposed to 2 years in the study described in WO 02/36157);
(d) the duration of treatment was long term (between 12 and 26 months); and
(e) the total number of patients in the trial was 3407 (as opposed to 220 in the study described in WO 02/36157). The study was a multicentre, multinational, IVRS-randomised, open-label, parallel-group study carried out across approximately 300 centres in approximately 25 countries.

Over the first 21 months of the trial, blood samples were taken from all patients in a standard manner at the following intervals: beforehand, and at, 1, 1.5, 2, 3, 4, 5, 6, 8, 10, 12, 15, 18, and 21, months. These samples were routinely analysed for total cholesterol, LDL (i.e. LDL-cholesterol), HDL (i.e. HDL-cholesterol), and total triglycerides, content using standard techniques for the detection of these lipids in serum.

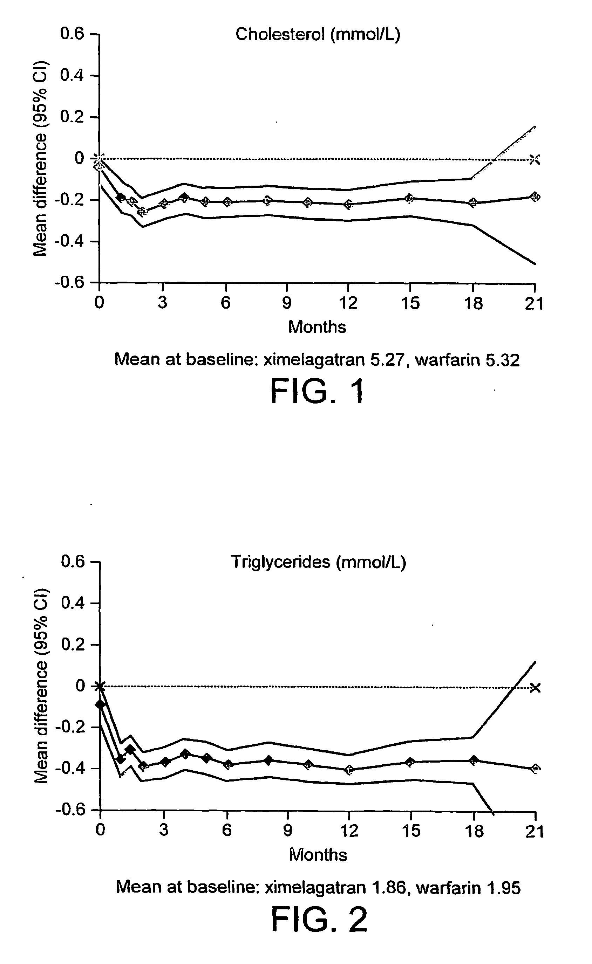

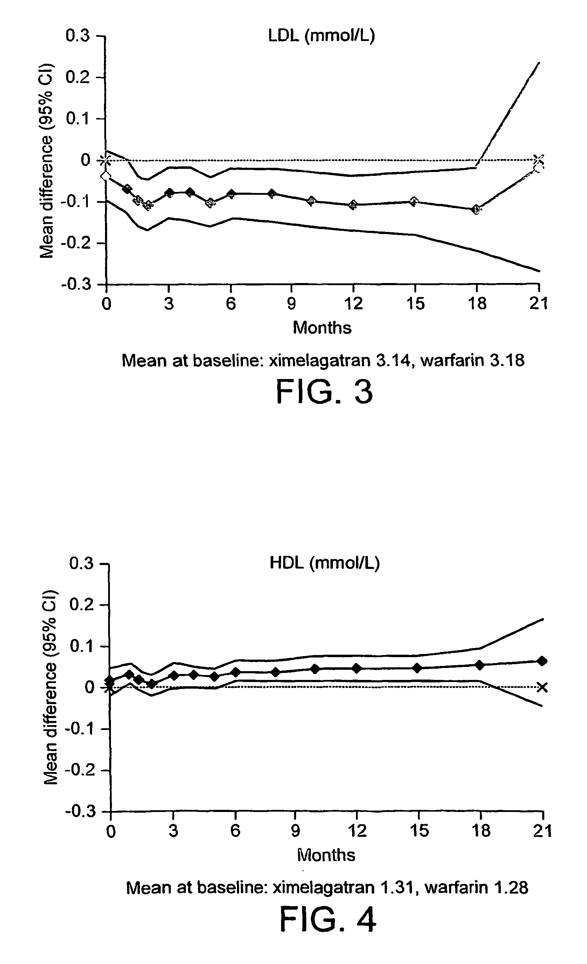

A comparison was made between patients on ximelagatran (n = 1704) and warfarin (n = 1703). When the raw data were analysed, an unexpected statistically-significant difference in favour of the ximelagatran group was observed. As of the second month of treatment, marked mean differences were observed for cholesterol, triglycerides and LDL serum concentrations (consistently significantly lower in the ximelagtran group over the entire 21 month period), and for the HDL serum concentration (consistently significantly higher in the ximelagtran group over the entire 21 month period), as illustrated in Figures 1 to 4, respectively.

These data clearly demonstrate the potential utility of melagatran and derivatives thereof as hereinbefore defined (e.g. prodrugs, such as ximelagtran), as well as, potentially, low molecular weight thrombin inhibitors and derivatives/prodrugs thereof, in cholesterol-lowering therapy.

## Claims

1. The use of melagatran or a pharmaceutically acceptable salt, solvate or compound of the formula
R¹O₂C-CH₂-(*R*)Cgl-(*S*)Aze-Pab-OH,
wherein Cgl represents cyclohexyl glycyl, Aze represents azetidine-2-carboxyl, Pab represents 4-amidinobenzylamino, R¹ represents linear or branched C₁₋₆ alkyl and the OH group replaces one of the amidino hydrogens in Pab, for the manufacture of a medicament for use in cholesterol-lowering therapy.

2. The use of melagatran or a pharmaceutically acceptable saft, solvate or compound of the formula
R¹O₂C-CH₂-(*R*)Cgl-*(S)*Aze-Pab-OH,
wherein Cgl represents cyclohexyl glycyl, Aze represents azetidine-2-carboxyl, Pab represents 4-amidinobenzylamino, R¹ represents linear or branched C₁₋₆ alkyl and the OH group replaces one of the amidino hydrogens in Pab, for the manufacture of a medicament for the treatment of hypercholesterolaemia, hyperlipoproteinaemia and/or hypertriglyceridaemia.

3. The use as claimed in Claim 1 or Claim 2, wherein the therapy/treatment results in a decrease in serum levels of cholesterol, low-density lipoproteins, very low-density lipoproteins, triglycerides and/or apolipoprotein B; and/or an increase in serum levels of high-density lipoproteins and/or apolipoprotein A-I.

4. The use as claimed in any one of Claims 1 to 3, wherein the use is of the compound of the formula
R¹O₂C-CH₂-(*R*)Cgl-(*S*)Aze-Pab-OH,
wherein R¹ represents linear or branched C₁₋₆ alkyl and the OH group replaces one of the amidino hydrogens in Pab

5. The use as claimed in any one of Claims 1 to 4. wherein R¹ represents methyl, ethyl or propyl..

6. The use as claimed in Claim 5, wherein R¹ represents ethyl.

7. The use of a thrombin inhibitor of formula I, 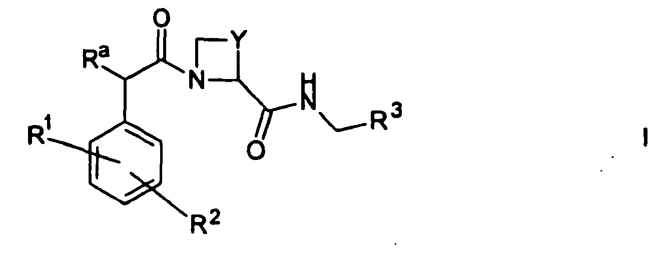 wherein
R^{a} represents -OH or -CH₂OH;
R¹ represents at least one optional halo substituent;
R² represents one or two C₁₋₃ alkoxy substituents, the alkyl parts of which substituents are themselves substituted with one or more fluoro substituents;
Y represents -CH₂- or -(CH₂)₂-; and
R³ represents a structural fragment of formula I(i) or I(ii): 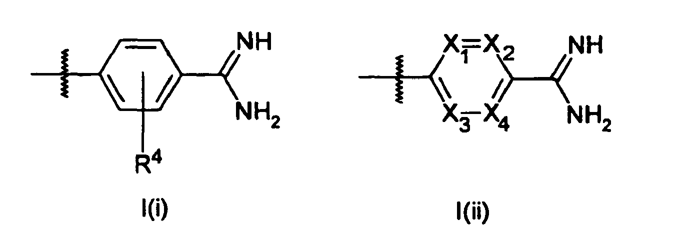 wherein
R⁴ represents H or one or more fluoro substituents; and
one or two of X₁, X₂, X₃ and X₄ represent -N- and the others represent -CH-, or a pharmaceutically acceptable salt or solvate thereof or a compound of formula Ia, 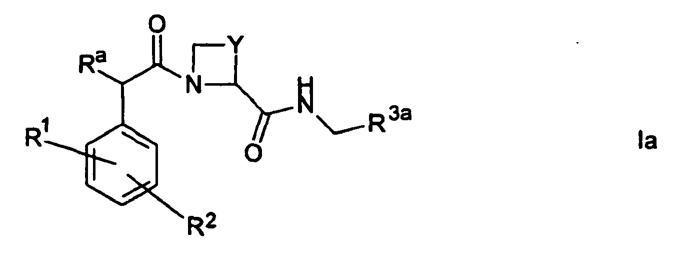
wherein R^{3a} represents a structural fragment of formula I(iii) or I(iv): 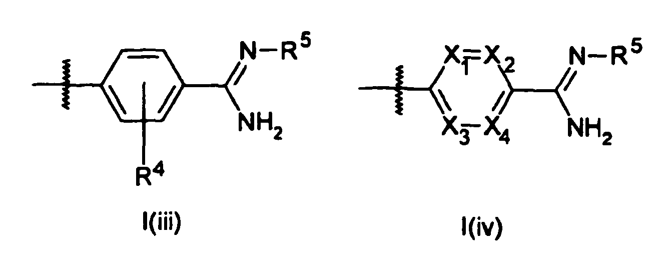
wherein R⁵ represents OR⁶ or C(O)OR⁷;
R⁶ represents H, C₁₋₁₀ alkyl, C₁₋₃ alkylaryl or C₁₋₃ alkyloxyaryl, the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents);
R⁷ represents C₁₋₁₀ alkyl, which latter group is optionally interrupted by one or more oxygen atoms, or C₁₋₃ alkylaryl or C₁₋₃ alkyloxyaryl, the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents; and
R^{a}, R¹, R², Y, R⁴, X₁, X₂, X₃ and X₄ are as defined above
for the manufacture of a medicament for use in cholesterol-lowering therapy.

8. The use of a thrombin inhibitor of formula I, as defined in Claim 7, a pharmaceutically acceptable salt or solvate thereof, or a compound of formula Ia as defined in Claim 7, for the manufacture of a medicament for the treatment of hypercholesterolaemia, hyperlipoproteinaemia and/or hypertriglyceridaemia.

9. The use as claimed in Claim 7 or Claim 8, wherein the therapy/treatment results in a decrease in serum levels of cholesterol, low-density lipoproteins, very low-density lipoproteins, Triglycerides and/or apolipoprotein B; and/or an increase in serum levels of high-density lipoproteins and/or apolipoprotein A-I.

10. The use as claimed in Claim 9, wherein the thrombin inhibitor is:
Ph(3-Cl)(5-OCHF₂)-(*R*)CH(OH)C(O)-(*S*)Aze-Pab-H,
Ph(3-Cl)(5-OCHF₂)-(*R*)CH(OH)C(O)-(*S*)Aze-Pab(2,6-diF); or
Ph(3-Cl)(5-OCH₂CH₂F)-(MCH(OH)C(O)-(*S*)Aze-Pab-H,
wherein Aze represents azetidine-2-carboxyl and Pab represents 4-amidinobenzylamino.

11. The use as claimed in any one of Claims 7 to 9, wherein the use is of a compound of formula Ia.

12. The use as claimed in Claim 11, wherein the compound is:
Ph(3-Cl)(5-OCHF₂)-(*R*)CH(OH)C(O)-(*S*)Aze-Pab(OMe);
Ph(3-Cl)(5-OCHF₂)-(*R*)CH(OH)C(O)-(*S*)Aze-Pab(2,6-diF)(OMe); or
Ph(3-Cl)(5-OCH₂CH₂F)-(*R*)CH(OH)C(O)-(*S*)Aze-Pab(OMe); wherein Aze represents azetidine-2-carboxyl and Pab represents 4-amidinobenzylamino.

13. A combination product comprising:
(A) melagatran, or a thrombin inhibitor of formula I as defined in Claim 7, or a pharmaceutically-acceptable salt or solvate thereof, a compound of the formula
R¹O₂C-CH₂-*(R)*Cgl-(*S*)Aze-Pab-OH.
wherein Cgl represents cyclohexylglycyl, Aze represents azetidine-2-carboxyl, Pab represents 4-amidinobenzylamino, R¹ represents linear or branched C₁₋₆ alkyl and the OH group replaces one of the amidino hydrogens in Pab, or a compound of formula Ia as defined in Claim 7; and
(B) another cholesterol-lowering, or lipid-lowering/modifying, therapeutic agent, wherein each of components (A) and (B) is formulated in admixture with a pharmaceutically-acceptable adjuvant, diluent or carrier.

14. A combination product as claimed in Claim 13, which comprises a pharmaceutical formulation including melagatran, or a thrombin inhibitor of formula I as defined in Claim 7, or salt or solvate thereof, or compound of the formula
R¹O₂C-CH₂-(*R*)Cgl-(*S*)Aze-Pab-OH,
wherein R¹ represents linear or branched C₁₋₆ alkyl and the OH group replaces one of the amidino hydrogens in Pab, or of formula Ia; the other therapeutic agent; and a pharmaceutically-acceptable adjuvant, diluent or carrier.

15. A combination product as claimed in Claim 13, which comprises a kit of parts comprising components:
(a) a pharmaceutical formulation including melagatran, a thrombin inhibitor of formula I as defined in Claim 7, or salt or solvate thereof or compound of the formula
R¹O₂C-CH₂-(*R*)Cgl-(*S*)Aze-Pab-OH,
wherein R¹ represents linear or branched C₁₋₆ alkyl and the OH group replaces one of the amidino hydrogens in Pab, or of formula Ia, in admixture with a pharmaceutically-acceptable adjuvant, diluent or carrier; and
(b) a pharmaceutical formulation including the other therapeutic agent in admixture with a pharmaceutically-acceptable adjuvant, diluent or carrier,
which components (a) and (b) are each provided in a form that is suitable for administration in conjunction with the other.

16. A combination product as claimed in any one of Claims 13 to 15, wherein component (A) is melagatran, or a pharmaceutically-acceptable salt or solvate thereof, or a compound of the formula
R¹O₂C-CH₂-(*R*)Cgl-(*S*)Aze-Pab-OH,
wherein R¹ represents linear or branched C₁₋₆ alkyl and the OH group replaces one of the amidino hydrogens in Pab.

17. A combination product as claimed in Claim 16, wherein component (A) is a compound of the formula
R¹O₂C-CH₂-(*R*)Cgl-(*S*)Aze-Pab-OH,
wherein R¹ represents linear or branched C₁₋₆ alkyl and the OH group replaces one of the amidino hydrogens in Pab.

18. A combination product as claimed in Claim 17, wherein R¹ represents methyl, ethyl or propyl.

19. A combination product as claimed in Claim 18, wherein R¹ represents ethyl.

20. A combination product as claimed in any one of Claims 13 to 15, wherein component (A) is a thrombin inhibitor of formula I as defined in Claim 7, or a pharmaceutically-acceptable salt or solvate thereof, or a compound of formula Ia as defined in Claim 7.

21. A combination product as claimed in Claim 20, wherein the thrombin inhibitor is:
Ph(3-Cl)(5-OCHF₂)-(*R*)CH(OH)C(O)-(*S*)AZe-Pab-H;
Ph(3-Cl)(5-OCHF₂)-(*R*)CH(OH)C(O)-(*S*)Aze-Pab(2,6-diF); or
Ph(3-Cl)(5-OCH₂CH₂F)-(*R*)CH(OH)C(O)-(*S*)Aze-Pab-H;

22. A combination product as claimed in Claim 20, wherein component (A) is a compound of formula Ia.

23. A combination product as claimed in Claim 22, wherein the compound is:
Ph(3-Cl)(5-OCHF₂)-(*R*)CH(OH)C(O)-(*S*)Aze-Pab(OMe);
Ph(3-Cl)(5-OCHF₂)-(*R*)CH(OH)C(O)-(*S*)Aze-Pab(2,6-diF)(OMe); or
Ph(3-Cl)(5-OCH₂CH₂F)-(*R*)CH(OH)C(O)-(*S*)Aze-Pab(OMe).

24. A combination product as claimed in any one of Claims 13 to 23, wherein the other therapeutic agent is a statin.

25. A combination product as claimed in Claim 24 wherein the statin is lovastatin, pravastatin, fluvastatin, simvastatin, atorvastatin, pitavastatin or rosuvastatin.

26. A compound as defined in any one of Claims 1 to 12 for use in cholesterol-lowering therapy.

27. A compound as defined in any one of Claims 1 to 12 for use in the treatment of hypercholesterolaemia, hyperlipoproteinaemia and/or hypertriglyceridaemia.

## Patentansprüche

1. Verwendung von Melagatran oder einem pharmazeutisch annehmbaren Salz oder Solvat oder einer Verbindung der Formel
R¹O₂C-CH₂-(*R*)Cgl-(*S*)Aze-Pab-OH
worin Cgl für Cyclohexylgycyl steht, Aze für Azetidin-2-carboxyl steht, Pab für 4-Amidinobenzylamino steht, R¹ für lineares oder verzweigtes C₁₋₆-Alkyl steht und die OH-Gruppe eines der Amidino-Wasserstoffatome in Pab ersetzt, zur Herstellung eines Arzneimittels zur Verwendung bei der cholesterinsenkenden Therapie.

2. Verwendung von Melagatran oder einem pharmazeutisch annehmbaren Salz oder Solvat oder einer Verbindung der Formel
R¹O₂C-CH₂-(*R*)Cgl-(*S*)Aze-Pab-OH
worin Cgl für Cyclohexylgycyl steht, Aze für Azetidin-2-carboxyl steht, Pab für 4-Amidinobenzylamino steht, R¹ für lineares oder verzweigtes C₁₋₆-Alkyl steht und die OH-Gruppe eines der Amidino-Wasserstoffatome in Pab ersetzt, zur Herstellung eines Arzneimittels zur Behandlung von Hypercholesterinämie, Hyperlipoproteinämie und/oder Hypertriglyceridämie.

3. Verwendung nach Anspruch 1 oder Anspruch 2, wobei die Therapie/Behandlung zu einer Verringerung der Serumspiegel von Cholesterin, Lipoproteinen niedriger Dichte, Lipoproteinen sehr niedriger Dichte, Triglyceriden und/oder Apolipoprotein B und/oder zu einer Erhöhung der Serumspiegel von Lipoproteinen hoher Dichte und/oder Apolipoprotein A-I führt.

4. Verwendung nach einem der Ansprüche 1 bis 3, wobei die Verwendung diejenige der Verbindung der Formel
R¹O₂C-CH₂-(*R*)Cgl-(*S*)Aze-Pab-OH
worin R¹ für lineares oder verzweigtes C₁₋₆-Alkyl steht und die OH-Gruppe eines der Amidino-Wasserstoffatome in Pab ersetzt, ist.

5. Verwendung nach einem der Ansprüche 1 bis 4, wobei R¹ für Methyl, Ethyl oder Propyl steht.

6. Verwendung nach Anspruch 5, wobei R¹ für Ethyl steht.

7. Verwendung eines Thrombininhibitors der Formel I 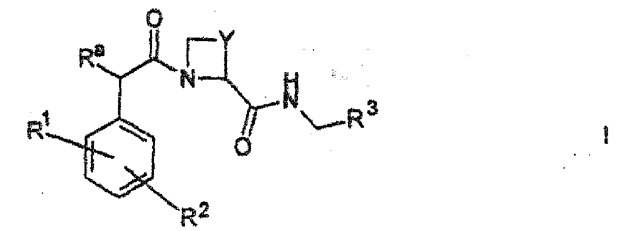 worin
R^{a} für -OH oder -CH₂OH steht;
R¹ für mindestens einen fakultativen Halogensubstituenten steht;
R² für einen oder zwei C₁₋₃-Alkoxysubstituenten steht, deren Alkylteile selbst durch einen oder mehrere Fluorsubstituenten substituiert sind;
Y für -CH₂- oder - (CH₂)₂- steht und
R³ für ein Strukturfragment der Formel I(i) oder I(ii) steht: 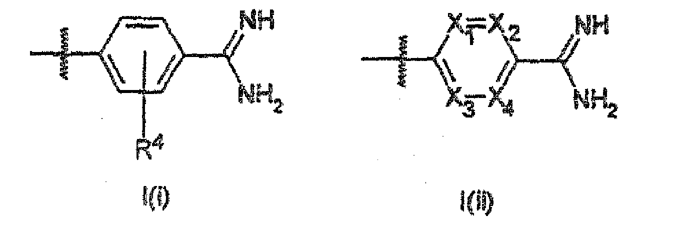 worin
R⁴ für H oder einen oder mehrere Fluorsubstituenten steht und
eine oder zwei der Gruppen X₁, X₂, X₃ und X₄ für -N-stehen und die anderen für -CH- stehen,
oder eines pharmazeutisch annehmbaren Salzes oder Solvats davon oder einer Verbindung der Formel Ia 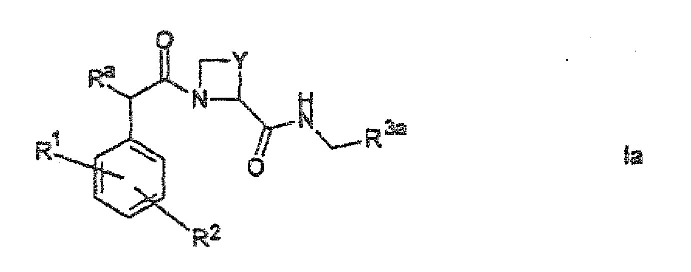
worin R^{3a} für ein Strukturfragment der Formel I(iii) oder I(iv) steht: 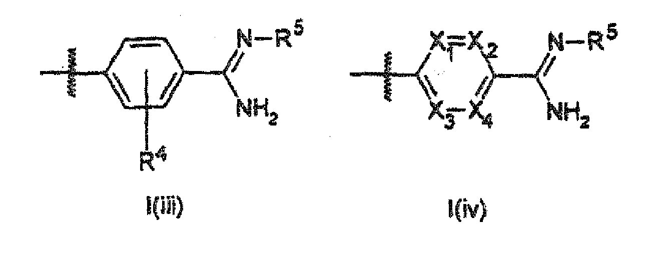
worin R⁵ für OR⁶ oder C(O)OR⁷ steht;
R⁶ für H, C₁₋₁₀-Alkyl, C₁₋₃-Alkylaryl oder C₁₋₃-Alkyloxyaryl steht, wobei die Alkylteile der letzteren beiden Gruppen gegebenenfalls durch ein oder mehrere Sauerstoffatome unterbrochen sind und die Arylteile der letzteren beiden Gruppen gegebenenfalls durch einen oder mehrere unter Halogen, Phenyl, Methyl oder Methoxy ausgewählte Substituenten substituiert sind, wobei die letzteren drei Gruppen auch gegebenenfalls durch einen oder mehrere Halogensubstituenten substituiert sind;
R⁷ für C₁₋₁₀-Alkyl, wobei die letztere Gruppe gegebenenfalls durch ein oder mehrere Sauerstoffatome unterbrochen ist, oder C₁₋₃-Alkylaryl oder C₁₋₃-Alkyloxyaryl steht, wobei die Alkylteile der letzteren beiden Gruppen gegebenenfalls durch ein oder mehrere Sauerstoffatome unterbrochen sind und die Arylteile der letzteren beiden Gruppen gegebenenfalls durch einen oder mehrere unter Halogen, Phenyl, Methyl oder Methoxy ausgewählte Substituenten substituiert sind, wobei die letzteren drei Gruppen auch gegebenenfalls durch einen oder mehrere Halogensubstituenten substituiert sind; und
R^{a}, R¹, R², Y, R⁴, X₁, X₂, X₃ und X₄ die oben angegebene Bedeutung besitzen,
zur Herstellung eines Arzneimittels zur Verwendung bei der cholesterinsenkenden Therapie.

8. Verwendung eines Thrombininhibitors der Formel I nach Anspruch 7, eines pharmazeutisch annehmbaren Salzes oder Solvats davon oder einer Verbindung der Formel Ia nach Anspruch 7 zur Herstellung eines Arzneimittels zur Behandlung von Hypercholesterinämie, Hyperlipoproteinämie und/oder Hypertriglyceridämie.

9. Verwendung nach Anspruch 7 oder Anspruch 8, wobei die Therapie/Behandlung zu einer Verringerung der Serumspiegel von Cholesterin, Lipoproteinen niedriger Dichte, Lipoproteinen sehr niedriger Dichte, Triglyceriden und/oder Apolipoprotein B und/oder zu einer Erhöhung der Serumspiegel von Lipoproteinen hoher Dichte und/oder Apolipoprotein A-I führt.

10. Verwendung nach Anspruch 9, wobei es sich bei dem Thrombininhibitor um:
Ph(3-Cl)(5-OCHF₂)-(*R*)CH(OH)C(O)-(*S*)Aze-Pab-H;
Ph(3-Cl)(5-OCHF₂)-(*R*)CH(OH)C(O)-(*S*)Aze-Pab(2,6-diF)
oder
Ph(3-Cl)(5-OCH₂CH₂F)-(*R*)CH(OH)C(O)-(*S*)Aze-Pab-H;
worin Aze für Azetidin-2-carboxyl steht und Pab für 4-Amidinobenzylamino steht,
handelt.

11. Verwendung nach einem der Ansprüche 7 bis 9, wobei die Verwendung diejenige der Verbindung der Formel Ia ist.

12. Verwendung nach Anspruch 11, wobei es sich bei der Verbindung um:
Ph(3-Cl)(5-OCHF₂)-(*R*)CH(OH)C(O)-(*S*)Aze-Pab(OMe);
Ph(3-Cl)(5-OCHF₂)-(*R*)CH(OH)C(O)-(*S*)Aze-Pab(2,6-diF) (OMe) oder
Ph(3-Cl)(5-OCH₂CH₂F)-(*R*)CH(OH)C(O)-(*S*)Aze-Pab(OMe);
worin Aze für Azetidin-2-carboxyl steht und Pab für 4-Amidinobenzylamino steht,
handelt.

13. Kombinationsprodukt, umfassend:
(A) Melagatran oder einen Thrombininhibitor der Formel I nach Anspruch 7 oder ein pharmazeutisch annehmbares Salz oder Solvat davon, eine Verbindung der Formel
R¹O₂C-CH₂-(*R*)Cgl-(*S*)Aze-Pab-OH
worin Cgl für Cyclohexylgycyl steht, Aze für Azetidin-2-carboxyl steht, Pab für 4-Amidinobenzylamino steht, R¹ für lineares oder verzweigtes C₁₋₆-Alkyl steht und die OH-Gruppe eines der Amidino-Wasserstoffatome in Pab ersetzt, oder einer Verbindung der Formel Ia nach Anspruch 7 und
(B) ein anderes cholesterinsenkendes oder lipidsenkendes/-modifizierendes Therapeutikum,
wobei jede der Komponenten (A) und (B) in Abmischung mit einem pharmazeutisch annehmbaren Hilfsstoff, Verdünnungsmittel oder Träger formuliert ist.

14. Kombinationsprodukt nach Anspruch 13, das eine pharmazeutische Formulierung mit Melagatran oder einem Thrombininhibitor der Formel I nach Anspruch 7 oder ein Salz oder Solvat davon oder eine Verbindung der Formel
R¹O₂C-CH₂-(*R*)Cgl-(*S*)Aze-Pab-OH
worin R¹ für lineares oder verzweigtes C₁₋₆-Alkyl steht und die OH-Gruppe eines der Amidino-Wasserstoffatome in Pab ersetzt, oder der Formel Ia, das andere Therapeutikum und einen pharmazeutisch annehmbaren Hilfsstoff, Verdünnungsmittel oder Träger umfaßt.

15. Kombinationsprodukt nach Anspruch 13, das ein Kit von Teilen umfaßt, die:
(a) eine pharmazeutische Formulierung, enthaltend Melagatran, einen Thrombininhibitor der Formel I nach Anspruch 7 oder ein Salz oder Solvat davon oder eine Verbindung der Formel
R¹O₂C-CH₂-(*R*)Cgl-(*S*)Aze-Pab-OH
worin R¹ für lineares oder verzweigtes C₁₋₆-Alkyl steht und die OH-Gruppe eines der Amidino-Wasserstoffatome in Pab ersetzt, oder der Formel Ia in Abmischung mit einem pharmazeutisch annehmbaren Hilfsstoff, Verdünnungsmittel oder Träger und
(b) eine pharmazeutische Formulierung, enthaltend das andere Therapeutikum in Abmischung mit einem pharmazeutisch annehmbaren Hilfsstoff, Verdünnungsmittel oder Träger
umfassen, wobei die Komponenten (a) und (b) jeweils in einer zur Verabreichung in Verbindung mit der anderen Komponente geeigneten Form bereitgestellt sind.

16. Kombinationsprodukt nach einem der Ansprüche 13 bis 15, wobei es sich bei Komponente (A) um Melagatran oder ein pharmazeutisch annehmbares Salz oder Solvat davon oder eine Verbindung der Formel
R¹O₂C-CH₂-(*R*)Cgl-(*S*)Aze-Pab-OH
worin R¹ für lineares oder verzweigtes C₁₋₆-Alkyl steht und die OH-Gruppe eines der Amidino-Wasserstoffatome in Pab ersetzt, handelt.

17. Kombinationsprodukt nach Anspruch 16, wobei es sich bei Komponente (A) um eine Verbindung der Formel
R¹O₂C-CH₂-(*R*)Cgl-(*S*)Aze-Pab-OH
worin R¹ für lineares oder verzweigtes C₁₋₆-Alkyl steht und die OH-Gruppe eines der Amidino-Wasserstoffatome in Pab ersetzt, handelt.

18. Kombinationsprodukt nach Anspruch 17, wobei R¹ für Methyl, Ethyl oder Propyl steht.

19. Kombinationsprodukt nach Anspruch 18, wobei R¹ für Ethyl steht.

20. Kombinationsprodukt nach einem der Ansprüche 13 bis 15, wobei es sich bei Komponente (A) um einen Thrombininhibitor der Formel I nach Anspruch 7 oder ein pharmazeutisch annehmbares Salz oder Solvat davon oder eine Verbindung der Formel Ia nach Anspruch 7 handelt.

21. Kombinationsprodukt nach Anspruch 20, wobei es sich bei dem Thrombininhibitor um:
Ph(3-Cl)(5-OCHF₂)-(*R*)CH(OH)C(O)-(*S*)Aze-Pab-H;
Ph(3-Cl)(5-OCHF₂)-(*R*)CH(OH)C(O)-(*S*)Aze-Pab(2,6-diF) oder
Ph(3-Cl)(5-OCH₂CH₂F)-(*R*)CH(OH)C(O)-(*S*)Aze-Pab-H handelt.

22. Kombinationsprodukt nach Anspruch 20, wobei es sich bei Komponente (A) um eine Verbindung der Formel Ia handelt.

23. Kombinationsprodukt nach Anspruch 22, wobei es sich bei der Verbindung um:
Ph(3-Cl)(5-OCHF₂)-(*R*)CH(OH)C(O)-(*S*)Aze-Pab(OMe);
Ph(3-Cl)(5-OCHF₂)-(*R*)CH(OH)C(O)-(*S*)Aze-Pab(2,6-diF) (OMe) oder
Ph(3-Cl)(5-OCH₂CH₂F)-(*R*)CH(OH)C(O)-(*S*)Aze-Pab(OMe) handelt.

24. Kombinationsprodukt nach einem der Ansprüche 13 bis 23, wobei es sich bei dem anderen Therapeutikum um ein Statin handelt.

25. Kombinationsprodukt nach Anspruch 24, wobei es sich bei dem Statin um Lovastatin, Pravastatin, Fluvastatin, Simvastatin, Atorvastatin, Pitavastatin oder Rosuvastatin handelt.

26. Verbindung nach einem der Ansprüche 1 bis 12 zur Verwendung bei der cholesterinsenkenden Therapie.

27. Verbindung nach einem der Ansprüche 1 bis 12 zur Verwendung bei der Behandlung von Hypercholesterinämie, Hyperlipoproteinämie und/oder Hypertriglyceridämie.

## Revendications

1. Utilisation de mélagatran ou d'un sel, solvate ou composé pharmaceutiquement acceptable de formule
R¹O₂C-CH₂-(*R*)Cgl-(*S*)Aze-Pab-OH,
dans laquelle Cgl représente cyclohexylglycyle, Aze représente azétidine-2-carboxyle, Pab représente 4-amidinobenzylamino, R¹ représente alkyle en C₁₋₆ linéaire ou ramifié et le groupement OH remplace l'un des hydrogènes de l'amidino dans Pab, pour la fabrication d'un médicament destiné à être utilisé en thérapie hypocholestérolémiante.

2. Utilisation de mélagatran ou d'un sel, solvate ou composé pharmaceutiquement acceptable de formule
R¹O₂C-CH₂-(*R*)Cgl-(*S*)Aze-Pab-OH,
dans laquelle Cgl représente cyclohexylglycyle, Aze représente azétidine-2-carboxyle, Pab représente 4-amidinobenzylamino, R¹ représente alkyle en C₁₋₆ linéaire ou ramifié et le groupement OH remplace l'un des hydrogènes de l'amidino dans Pab, pour la fabrication d'un médicament destiné au traitement de l'hypercholestérolémie, de l'hyperlipoprotéinémie et/ou de l'hypertriglycéridémie.

3. Utilisation selon la revendication 1 ou la revendication 2, **caractérisée en ce que** la thérapie/le traitement conduit à une baisse des taux sériques de cholestérol, des lipoprotéines de faible densité, des lipoprotéines, des triglycérides et/ou de l'apolipoprotéine B de très faible densité; et/ou une augmentation des taux sériques des lipoprotéines et/ou de l'apolipoprotéine A-I de haute densité.

4. Utilisation selon l'une quelconque des revendications 1 à 3, **caractérisée en ce qu'**il s'agit de l'utilisation du composé de formule
R¹O₂C-CH₂-(*R*)Cgl-(*S*)Aze-Pab-OH,
dans laquelle R¹ représente alkyle en C₁₋₆ linéaire ou ramifié et le groupement OH remplace l'un des hydrogènes de l'amidino dans Pab.

5. Utilisation selon l'une quelconque des revendications 1 à 4, **caractérisée en ce que** R¹ représente méthyle, éthyle ou propyle.

6. Utilisation selon la revendication 5 **caractérisée en ce que** R¹ représente éthyle.

7. Utilisation d'un inhibiteur de la thrombine de formule I, 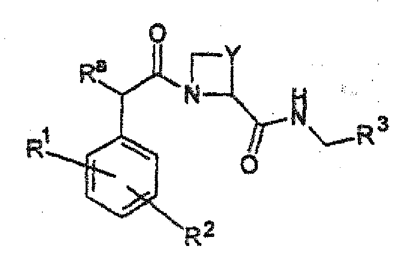 dans laquelle
R^{a} représente -OH ou -CH₂OH;
R¹ représente au moins un substituant halogéno éventuel;
R² représente un ou deux substituants alcoxy en C₁₋₃₁ les parties alkyle desdits substituants étant elles-mêmes substituées par un ou plusieurs substituants fluoro;
Y représente -CH₂- ou -(CH₂)₂-; et
R³ représente un fragment structural de formule I(i) ou I (ii) : 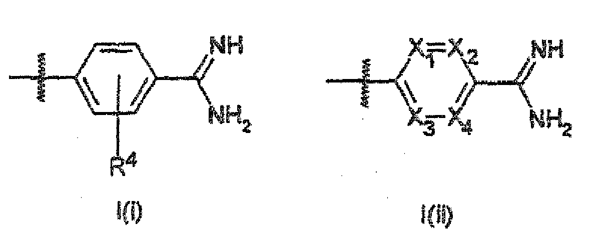 dans lesquelles
R⁴ représente H ou un ou plusieurs substituants fluoro; et
un ou deux de X₁, X₂, X₃ et X₄ représentent -N- et les autres représentent -CH-, ou d'un sel ou solvate pharmaceutiquement acceptable de celui-ci ou d'un composé de formule Ia, 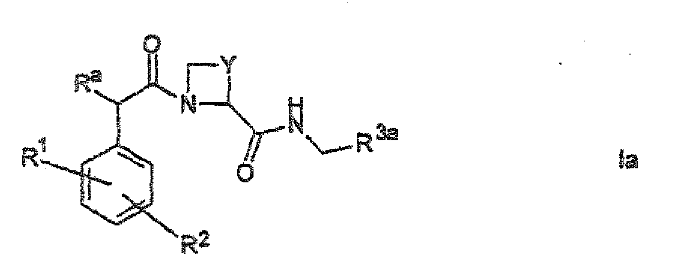
dans laquelle R^{3a} représente un fragment structural de formule I(iii) ou I(iv): 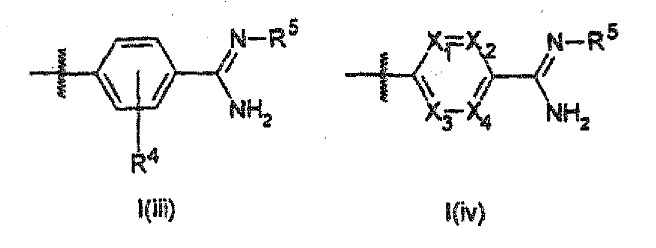
dans lesquelles R⁵ représente OR⁶ ou C(O)OR⁷;
R⁶ représente H, alkyle en C₁₋₁₀, alkylaryle en C₁₋₃ ou alkyloxyaryle en C₁₋₃, les parties alkyle desdits deux derniers groupements étant éventuellement interrompues par un ou plusieurs atomes d'oxygène, et les parties aryle desdits deux derniers groupements étant éventuellement substituées par un ou plusieurs substituants choisis parmi halogéno, phényle, méthyle ou méthoxy, lesdits trois derniers groupements étant également éventuellement substitués par un ou plusieurs substituants halogéno;
R⁷ représente alkyle en C₁₋₁₀, ledit dernier groupement étant éventuellement interrompu par un ou plusieurs atomes d'oxygène, ou alkylaryle en C₁₋₃ ou alkyloxyaryle en C₁₋₃, les parties alkyle desdits deux derniers groupements étant éventuellement interrompues par un ou plusieurs atomes d'oxygène, et les parties aryle desdits deux derniers groupements étant éventuellement substituées par un ou plusieurs substituants choisis parmi halogéno, phényle, méthyle ou méthoxy, lesdits trois derniers groupements étant également éventuellement substitués par un ou plusieurs substituants halogéno; et
R^{a}, R¹, R², Y, R⁴, X₁, X₂, X₃ et X₄ sont tels que définis ci-dessus
pour la fabrication d'un médicament destiné à être utilisé en thérapie hypocholestérolémiante.

8. Utilisation d'un inhibiteur de la thrombine de formule I, tel que défini dans la revendication 7, d'un sel ou solvate pharmaceutiquement acceptable de celui-ci, ou d'un composé de formule la tel que défini dans la revendication 7, pour la fabrication d'un médicament destiné au traitement de l'hypercholestérolémie, de l'hyperlipoprotéinémie et/ou de l'hypertriglycéridémie.

9. Utilisation selon la revendication 7 ou la revendication 8, **caractérisée en ce que** la thérapie/le traitement conduit à une baisse des taux sériques de cholestérol, des lipoprotéines de faible densité, des lipoprotéines, des triglycérides et/ou de l'apolipoprotéine B de très faible densité; et/ou une augmentation des taux sériques des lipoprotéines et/ou de l'apolipoprotéine A-I de haute densité.

10. Utilisation selon la revendication 9 **caractérisée en ce que** l'inhibiteur de la thrombine est:
Ph (3-Cl) (5-OCHF₂) - (*R*)CH(OH)C(O) - (*S*) Aze-Pab-H,
Ph (3-Cl) (5-OCHF₂)-(*R*)CH(OH)C(O)*-*(*S*)Aze-Pab(2,6-diF) ; ou
Ph(3-Cl) (5-OCH₂CH₂F) - (*R*)CH(OH)C(O) - (*S*)Aze-Pab-H, où Aze représente azétidine-2-carboxyle et Pab représente 4-amidinobenzylamino.

11. Utilisation selon l'une quelconque des revendications 7 à 9, **caractérisée en ce qu'**il s'agit de l'utilisation d'un composé de formule Ia.

12. Utilisation selon la revendication 11, **caractérisée en ce que** le composé est:
Ph(3-Cl) (5-OCHF₂)-(*R*)CH(OH)C(O)-(*S*)Aze-Pab(OMe) ;
Ph(3-Cl) (5-OCHF2)-(*R*)CH(OH)C(O)-(*S*)Aze-Pab(2,6-diF) (OMe) ; ou
Ph(3-Cl) (5-OCH₂CH₂F) - (*R*)CH(OH)C(O) - (*S*)Aze-Pab(OMe) , où Aze représente azétidine-2-carboxyle et Pab représente 4-amidinobenzylamino.

13. Produit combiné comprenant:
(A) le mélagatran, ou un inhibiteur de la thrombine de formule I tel que défini dans la revendication 7, ou un sel ou solvate pharmaceutiquement acceptable de celui-ci, un composé de formule
R¹O₂C-CH₂-(*R*)Cgl-(*S*)Aze-Pab-OH,
dans laquelle Cgl représente cyclohexylglycyle, Aze représente azétidine-2-carboxyle, Pab représente 4-amidinobenzylamino, R¹ représente alkyle en C₁₋₆ linéaire ou ramifié et le groupement OH remplace l'un des hydrogènes de l'amidino dans Pab, ou un composé de formule la tel que défini dans la revendication 7; et
(B) un autre agent thérapeutique hypocholestérolémiant ou abaissant/modifiant le taux des lipides,
chacun des composants (A) et (B) étant formulé en mélange avec un adjuvant, un diluant ou un support pharmaceutiquement acceptable.

14. Produit combiné selon la revendication 13, **caractérisé en ce qu'**il comprend une formulation pharmaceutique comportant le mélagatran, ou un inhibiteur de la thrombine de formule I tel que défini dans la revendication 7, ou un sel ou solvate de celui-ci ou un composé de formule
R¹O₂C-CH₂- (*R*) Cgl- (*S*)Aze-Pab-OH,
dans laquelle R¹ représente alkyle en C₁₋₆ linéaire ou ramifié et le groupement OH remplace l'un des hydrogènes de l'amidino dans Pab, ou de formule Ia; l'autre agent thérapeutique; et un adjuvant, un diluant ou un support pharmaceutiquement acceptable.

15. Produit combiné selon la revendication 13, **caractérisé en ce qu'**il comprend un kit de parties comprenant des composants:
(a) une formulation pharmaceutique comportant le mélagatran, un inhibiteur de la thrombine de formule I tel que défini dans la revendication 7, ou un sel ou solvate de celui-ci ou un composé de formule
R¹O₂C-CH₂- (*R*) Cgl- (*S*)Aze-Pab-OH,
dans laquelle R¹ représente alkyle en C₁₋₆ linéaire ou ramifié et le groupement OH remplace l'un des hydrogènes de l'amidino dans Pab, ou de formule Ia, en mélange avec un adjuvant, un diluant ou un support pharmaceutiquement acceptable, et
(b) une formulation pharmaceutique comportant l'autre agent thérapeutique en mélange avec un adjuvant, un diluant, ou un support pharmaceutiquement acceptable,
lesquels composants (a) et (b) sont chacun fournis sous une forme qui est adaptée à l'administration conjointement avec l'autre.

16. Produit combiné selon l'une quelconque des revendications 13 à 15, **caractérisé en ce que** le composant (A) est le mélagatran ou un sel ou un solvate pharmaceutiquement acceptable de celui-ci, ou un composé de formule
R¹O₂C-CH₂- (*R*) Cgl- (*S*)Aze-Pab-OH,
dans laquelle R¹ représente alkyle en C₁₋₆ linéaire ou ramifié et le groupement OH remplace l'un des hydrogènes de l'amidino dans Pab.

17. Produit combiné selon la revendication 16, **caractérisé en ce que** le composant (A) est un composé de formule
R¹O₂C-CH₂- (*R*) Cgl- (*S*)Aze-Pab-OH,
dans laquelle R¹ représente alkyle en C₁₋₆ linéaire ou ramifié et le groupement OH remplace l'un des hydrogènes de l'amidino dans Pab.

18. Produit combiné selon la revendication 17, **caractérisé en ce que** R¹ représente méthyle, éthyle ou propyle.

19. Produit combiné selon la revendication 18, **caractérisé en ce que** R¹ représente éthyle.

20. Produit combiné selon l'une quelconque des revendications 13 à 15, **caractérisé en ce que** le composant (A) est un inhibiteur de la thrombine de formule I tel que défini dans la revendication 7 ou un sel ou solvate pharmaceutiquement acceptable de celui-ci, ou un composé de formule la tel que défini dans la revendication 7.

21. Produit combiné selon la revendication 20, **caractérisé en ce que** l'inhibiteur de la thrombine est:
Ph (3-Cl) (5-OCHF₂) - (*R*)CH(OH)C(O) - (*S*)Aze-Pab-H,
Ph(3-Cl) (5-OCHF₂)-(*R*)CH(OH)C(O)-(*S*)Aze-Pab(2,6-diF) ; ou
Ph (3-Cl) (5-OCH₂CH₂F) - (*R*)CH(OH)C(O) - (*S*)Aze-Pab-H.

22. Produit combiné selon la revendication 20, **caractérisé en ce que** le composant (A) est un composé de formule Ia.

23. Produit combiné selon la revendication 22, **caractérisé en ce que** le composé est:
Ph(3-Cl) (5-OCHF₂) - (*R*)CH(OH)C(O) - (*S*)Aze-Pab(OMe) ;
Ph (3 - Cl) (5-OCHF₂) - (*R*)CH(OH)C(O) - (*S*)Aze-Pab(2,6-diF) (OMe) ; ou
Ph(3-Cl) (5-OCH₂CH₂F) - (*R*)CH(OH)C(O) - (*S*)Aze-Pab(OMe).

24. Produit combiné selon l'une quelconque des revendications 13 à 23, **caractérisé en ce que** l'autre agent thérapeutique est une statine.

25. Produit combiné selon la revendication 24, **caractérisé en ce que** la statine est la lovastatine, la pravastatine, la fluvastatine, la simvastatine, l'atorvastatine, la pitavastatine ou la rosuvastatine.

26. Composé tel que défini dans l'une quelconque des revendications 1 à 12, destiné à être utilisé en thérapie hypocholestérolémiante.

27. Composé tel que défini dans l'une quelconque des revendications 1 à 12, destiné à être utilisé dans le traitement de l'hypercholestérolémie, de l'hyperlipoprotéinémie et/ou de l'hypertriglycéridémie.
